# EUROPÄISCHE PATENTANMELDUNG

(11) **EP 4 559 400 A1**
(43) Veröffentlichungstag der Anmeldung: **28.05.2025**
(21) Anmeldenummer: 24212696.9
(22) Anmeldetag: 13.11.2024
(51) Int. Cl.: A61B 6/00

(54) **REINIGUNGSEINHEIT ZUM REINIGEN EINER EINEN PATIENTENAUFNAHMEBEREICH UMGEBENDEN UMHAUSUNG**

(30) Priorität: 24.11.2023 AT 509492023
(71) Anmelder: Hagleitner, Hans Georg, 5700 Zell am See (AT)
(72) Erfinder: Hagleitner, Hans Georg, 5700 Zell am See (AT)
(74) Vertreter: Torggler & Hofmann Patentanwälte - Innsbruck

(57) **Zusammenfassung**

Reinigungseinheit (1) zum Reinigen einer einen Patientenaufnahmebereich (2) umgebenden Umhausung (3) einer medizinischen Bildgebungsvorrichtung (4), mit einer Trägerstruktur (5) und mit einer Reinigungskomponente (6), wobei die Reinigungskomponente (6) der in die Umhausung (3) eingeführten Reinigungseinheit (1) zwischen der Trägerstruktur (5) und der Umhausung (3) angeordnet ist, wobei die Reinigungskomponente (6) in einem mit einem Druckmedium befüllten Zustand an der Umhausung (3), bevorzugt pressend, anliegt.

## Beschreibung

Die vorliegende Erfindung betrifft
- eine Reinigungseinheit zum Reinigen einer einen Patientenaufnahmebereich umgebenden Umhausung einer medizinischen Bildgebungsvorrichtung, mit einer Trägerstruktur und mit einer Reinigungskomponente,
- eine medizinische Bildgebungsvorrichtung mit einer solchen Reinigungseinheit und
- ein Verfahren zum Reinigen einer solchen medizinischen Bildgebungsvorrichtung.

Medizinischen Bildgebungsvorrichtungen werden in der Radiologie verwendet und sind zum Beispiel MRT- und CT-Geräte. Diese werden bei Verwendung verschmutzt und/oder mit Keimen und/oder Viren kontaminiert. Dies kann beispielsweise durch die zu untersuchenden Personen selbst innerhalb der medizinischen Bildgebungsvorrichtung erfolgen, wenn bei Husten, Niesen, feuchter Atmung, physischer Berührung der medizinischen Bildgebungsvorrichtung oder Ähnlichem Keime oder andere Verschmutzungen hinterlassen werden. Derartige Verschmutzungen verbleiben unter anderem auf der Innenseite, konkret einer Umhausung, der medizinischen Bildgebungsvorrichtung.

Eine manuelle Reinigung erfolgt hierbei mit einem optional an einer Stange befestigen Reinigungsutensil, wie beispielsweise in US 5918342 offenbart.

Nachteilig dabei ist, dass diese Art der Reinigung sehr umständlich ist und/oder sich das Reinigungspersonal sehr nahe oder sogar teilweise innerhalb der medizinischen Bildgebungsvorrichtung aufhält. Der radiologische Einfluss, wie zum Beispiel Röntgenstrahlung oder Magnetfelder, der medizinischen Bildgebungsvorrichtung kann dabei unangenehm sein.

Es ist weiters bekannt, automatische Reinigungseinheiten innerhalb der medizinischen Bildgebungsvorrichtung vorzusehen, wie beispielsweise in CN 219251060 U oder DE 102021210471 A1 offenbart. Dabei kann die Reinigungseinheit eine Wischreinigung durchführen, indem die Reinigungseinheit von einem Ende der Umhausung zum anderen Ende der Umhausung verfahren wird.

Nachteilig dabei ist, dass eine derartige Reinigungseinheit nach einem ersten Verfahren der Reinigungseinheit und Wischen der Umhausung beim Zurückfahren in die Ausgangsposition die Umhausung zumindest teilweise rekontaminiert, da die beim ersten Verfahren aufgenommenen Keime und/oder Viren wieder auf der Umhausung hinterlassen werden können. Außerdem muss beim Einführen einer derartigen Reinigungseinheit in die Umhausung einer Medizinischen Bildgebungsvorrichtung, die Positionierung und die geometrische Genauigkeit der Reinigungseinheit beim aktuellen Stand der Technik entweder sehr exakt sein oder ein aufwändiger Mechanismus für den Ausgleich von Toleranzen vorgesehen sein. Diesbezügliche Abweichungen und/oder Ungenauigkeiten wirken sich negativ auf die Reinigungsleistung aus.

Die Aufgabe der vorliegenden Erfindung besteht also darin, die Nachteile des Standes der Technik zumindest teilweise zu beheben und eine gegenüber dem Stand der Technik verbesserte Reinigungseinheit anzugeben, welche sich insbesondere durch eine zuverlässige Reinigung von medizinischen Bildgebungsvorrichtungen auszeichnet. Die Aufgabe besteht weiterhin darin, eine medizinische Bildgebungsvorrichtung mit einer solchen Reinigungseinheit und ein Verfahren zum Reinigen einer solchen medizinischen Bildgebungsvorrichtung anzugeben.

Diese Aufgabe wird gelöst durch die Merkmale der Ansprüche 1, 2, 11 und 14.

Diese Aufgabe wird gemäß einem ersten Aspekt der Erfindung mittels einer Reinigungseinheit nach Anspruch 1 gelöst, nämlich einer Reinigungseinheit zum Reinigen einer einen Patientenaufnahmebereich umgebenden Umhausung einer medizinischen Bildgebungsvorrichtung, mit einer Trägerstruktur und mit einer Reinigungskomponente, wobei die Reinigungskomponente der in die Umhausung eingeführten Reinigungseinheit zwischen der Trägerstruktur und der Umhausung angeordnet ist, wobei die Reinigungskomponente in einem mit einem Druckmedium befüllten Zustand an der Umhausung, bevorzugt pressend, anliegt.

Unter "Reinigen" wird hier Säubern, Desinfizieren und/oder Sterilisieren verstanden. Das Reinigen kann beispielsweise in Form von Wischen, Abschaben, Abkratzen, Reiben, Schrubben, Waschen und/oder dergleichen erfolgen.

Durch einen mit einem Druckmedium befüllten Zustand der Reinigungskomponente wird ein Anliegen der Reinigungskomponente an der Umhausung gewährt. In einem nicht-befüllten Zustand liegt die Reinigungskomponente nicht an der Umhausung an. Je nach Druck des Druckmediums, mit dem die Reinigungskomponente befüllt ist, kann der Druck des Anliegens gesteuert werden. Ein geringerer Druck des Druckmediums führt zu einem schwach angepressten Anliegen, wohingegen ein höherer Druck des Druckmediums zu einem stark angepressten Anliegen führt.

Diese Aufgabe wird gemäß einem zweiten Aspekt der Erfindung mittels einer Reinigungseinheit nach Anspruch 2 gelöst, nämlich einer Reinigungseinheit zum Reinigen einer einen Patientenaufnahmebereich umgebenden Umhausung einer medizinischen Bildgebungsvorrichtung, mit einer Trägerstruktur und mit einer Reinigungskomponente, wobei die Reinigungskomponente entlang einer, bevorzugt äußeren, Kontur der Trägerstruktur angeordnet ist, wobei die Reinigungskomponente durch Befüllen mit einem Druckmedium verformbar ist.

Unter "verformbar" wird hier verstanden, dass die Form eines Körper infolge der Einwirkung einer Kraft und/oder einer Spannung verändert werden kann. Eine Verformung eines Körper kann in einen ersten Anteil, bei dem sich das Volumen und/oder die räumliche Ausdehnung des Körpers aber nicht die geometrische Form des Körpers ändert, beispielsweise bei einer Radiusvergrößerung einer Kugel oder eines schlauchförmigen Körpers, und in einen zweiten Anteil, bei dem sich die geometrische Form des Körpers aber nicht das Volumen und/oder die räumliche Ausdehnung des Körpers ändert, beispielsweise bei einer Dehnung einer Kugel entlang einer Achse zu einem Ellipsoid, zerlegt werden. Bei einer Verformung durch Befüllung der Reinigungskomponente mit einem Druckmedium kann eine Verformung mit einem ersten und/oder einem zweiten Anteil erfolgen.

Unter "durch Befüllen mit einem Druckmedium verformbare Reinigungskomponente" kann hier verstanden werden, dass eine Reinigungskomponente verformbar ist, indem ein teilweise, bevorzugt vollständig, durch die Reinigungskomponente bestimmtes Volumen zumindest teilweise, bevorzugt vollständig, durch ein Druckmedium eingenommen wird.

Die Verformbarkeit der Reinigungskomponente durch Befüllen mit einem Druckmedium bewirkt, dass eine geometrische Form, bevorzugt ein Außendurchmesser, die räumliche Ausdehnung und/oder das Volumen der Reinigungskomponente geändert wird. Auf diese Weise kann ein Kontaktieren der Reinigungseinheit mit der Umhausung der medizinischen Bildgebungsvorrichtung gezielt hervorgerufen oder unterlassen werden.

Durch die beiden Aspekte der Erfindung wird eine zuverlässige Reinigung einer Umhausung durchgeführt, indem die Reinigungskomponente in einen befüllten Zustand versetzt wird, die Umhausung durch die befüllte Reinigungskomponente kontaktiert wird und die Reinigungskomponente beim Bewegen der Reinigungseinheit die Umhausung beispielsweise durch Wischen reinigt. Sobald das Bewegen der Reinigungseinheit entlang der Umhausung in eine erste Richtung beendet ist, kann die befüllte Reinigungskomponente in einen nicht-befüllten Zustand versetzt werden und die Reinigungseinheit mit der nicht-befüllten Reinigungskomponente kontaktlos wieder zurück in die Ausgangsposition bewegt werden. Es kommt dabei zu keinem erneuten Kontakt der Reinigungskomponente mit der Umhausung, womit eine Rekontamination vermieden wird.

Eine "nicht-befüllte Reinigungskomponente" oder eine "Reinigungskomponente in einem nicht-befüllten Zustand" ist eine Reinigungskomponente, welche nicht mit einem Druckmedium befüllt ist, wobei die nicht-befüllte Reinigungskomponente einer in die Umhausung eingeführten Reinigungseinheit die Umhausung nicht kontaktiert.

In einem nicht-befüllten Zustand steht die Reinigungskomponente unter der Kraft- und/oder Spannungseinwirkung des die Reinigungskomponente umgebenden Atmosphärendrucks und optional eines in der Reinigungskomponente vorhandenen Mediums, welches nicht das Druckmedium ist, aber keinesfalls unter der Druckwirkung der Umhausung.

Eine "teilweise befüllte Reinigungskomponente" oder eine "Reinigungskomponente in einem teilweise befüllten Zustand" ist eine Reinigungskomponente, welche zumindest teilweise mit einem Druckmedium befüllt ist, wobei die teilweise befüllte Reinigungskomponente einer in die Umhausung eingeführten Reinigungseinheit die Umhausung nicht kontaktiert.

Eine "befüllte Reinigungskomponente" oder eine "Reinigungskomponente in einem befüllten Zustand" ist eine Reinigungskomponente, welche mit einem Druckmedium befüllt ist, wobei die befüllte Reinigungskomponente einer in die Umhausung eingeführten Reinigungseinheit die Umhausung kontaktiert.

In einem befüllten Zustand steht die Reinigungskomponente unter der Kraft- und/oder Spannungseinwirkung des Druckmediums, des die Reinigungskomponente umgebenden Atmosphärendrucks und der die Reinigungskomponente kontaktierenden Umhausung und optional einer Reaktionskraft der, bevorzugt elastischen, Reinigungskomponente infolge der Verformung.

Beim Übergang einer befüllten Reinigungskomponente, bei der ein Kontaktieren der Umhausung vorliegt, über eine teilweise befüllte Reinigungskomponente bis hin zu einer nicht-befüllten Reinigungskomponente wird Druckmedium aus der Reinigungskomponente entnommen, wodurch sich eine räumliche Ausdehnung der Reinigungskomponente verringert und das Kontaktieren der Umhausung beendet wird.

Beim Übergang einer nicht-befüllten Reinigungskomponente, bei der kein Kontaktieren der Umhausung vorliegt, über eine teilweise befüllte Reinigungskomponente bis hin zu einer befüllten Reinigungskomponente wird Druckmedium in die Reinigungskomponente eingeführt, wodurch sich eine räumliche Ausdehnung der Reinigungskomponente vergrößert und das Kontaktieren der Umhausung erreicht wird.

Erst wenn ausreichend Druckmedium in eine Reinigungskomponente einer Reinigungseinheit eingeführt ist oder, mit anderen Worten, wenn die Reinigungskomponente ausreichend mit Druckmedium befüllt ist, findet der Übergang von der teilweise befüllten Reinigungskomponente hin zur befüllten Reinigungskomponente statt, wodurch ein Kontaktieren der Umhausung durch eine in die Umhausung eingeführten Reinigungseinheit ermöglicht wird. Wird anschließend eine bereits befüllte Reinigungskomponente noch weiter mit Druckmedium befüllt, so liegt ein Kontaktieren der Umhausung in Form eines Anpressens vor, wobei der Anpressdruck durch den Grad der Befüllung der befüllten Reinigungskomponente bestimmt wird.

Eine Reinigungskomponente kann mehrmals hintereinander befüllt werden und bei jeder neuen Befüllung seine räumliche Ausdehnung entsprechend obigen Ausführungen vergrößern. Dies gilt analog für eine Reinigungskomponente, der mehrmals hintereinander ein Teil des Druckmediums entnommen wird.

Ein weiterer Vorteil ist, dass die befüllte Reinigungskomponente alle Spalten zwischen der Reinigungseinheit und der Umhausung schließen kann, womit ein lückenfreies Reinigen, beispielsweise Wischen, ermöglicht werden kann.

Weitere Vorteile sind:
- Ein gleichmäßiger Anpressdruck der befüllten Reinigungskomponente auf die Umhausung kann realisiert werden.
- Fertigungstoleranzen der Umhausung und der Positionierung einer Verfahrvorrichtung, beispielsweise eines beweglichen Patiententisches und/oder einer Patiententischführung zur und/oder innerhalb der Umhausung können ausgeglichen werden.
- Es können unterschiedliche Abmessungen, bevorzugt Innendurchmesser, von verschiedenartigen Umhausungen unterschiedlicher Modelle von medizinischen Bildgebungsvorrichtungen ausgeglichen werden.
- Einfache Bereitstellung eines Spalts zwischen Reinigungskomponente und Umhausung beim Bewegen der Reinigungskomponente nach dem Reinigen der Umhausung.
- Vorbereitungsarbeiten oder Wartungsarbeiten wie beispielsweise das Wechseln von Reinigungseinheiten oder einzelnen Teilen der Reinigungseinheit kann immer von der gleichen Seite der medizinischen Bildgebungsvorrichtungen durchgeführt werden.

Der Vollständigkeit halber wird darauf hingewiesen, dass im Zuge der Beschreibung dieser Erfindung die verwendeten Zahlwörter wie ein, zwei, drei und dergleichen, grundsätzlich nur die vorhandene Mindestmenge eines Merkmals der erfindungsgemäßen Reinigungseinheit, der erfindungsgemäßen medizinischen Bildgebungsvorrichtung mit einer solchen Reinigungseinheit und/oder des erfindungsgemäßen Verfahrens zum Reinigen einer solchen medizinischen Bildgebungsvorrichtung beschreibt. Einzelne Merkmale oder Bestandteile können natürlich auch in größerer Anzahl vorhanden sein. So können beispielsweise erfindungsgemäße Reinigungseinheit mehr als eine Reinigungskomponente, mehr als eine Trägerstruktur usw. aufweisen. In diesem Sinne ist also beispielsweise das Zahlwort ein soweit sinnvoll im Sinne von mindestens ein usw. zu verstehen.

Weitere vorteilhafte Ausführungsformen der Anordnung werden in den abhängigen Ansprüchen definiert.

Gemäß einer bevorzugten Ausführungsform der Reinigungseinheit ist es vorgesehen, dass eine äußere Kontur der Trägerstruktur und/oder der, bevorzugt befüllten, Reinigungskomponente korrespondierend zu einer inneren Kontur der Umhausung und bevorzugt gebogen, besonders bevorzugt kreisförmig, ist.

In einer bevorzugten Ausführungsvariante kann vorgesehen sein, dass eine äußere Kontur der nicht-befüllten Reinigungskomponente korrespondierend zu einer inneren Kontur der Umhausung ist, wobei ein Spalt zwischen den Konturen vorgesehen ist.

Mit anderen Worten, wenn eine Kontur zu einer anderen Kontur korrespondierend ist, dann folgt die eine Kontur der anderen Kontur.

Durch die zueinander korrespondierenden und bevorzugt gebogenen, besonders bevorzugt kreisförmigen, Konturen der Trägerstruktur und/oder der Reinigungskomponente und der Umhausung kann ein spaltfreies Kontaktieren noch zuverlässiger erreicht werden, da die befüllte Reinigungskomponente kaum Formabweichungen ausgleichen muss.

In einer bevorzugten Ausführungsvariante kann vorgesehen sein, dass die Trägerstruktur formstabil ist und/oder die Reinigungskomponente elastisch verformbar ist.

Durch die elastische Verformbarkeit der Reinigungskomponente kann ein höherer Druck des Druckmediums und somit eine größere räumliche Ausdehnung erreicht werden. Somit kann sich die Reinigungskomponente noch bessere und flexibler an die Umhausung anpassen und/oder dichter angepresst werden.

In einer bevorzugten Ausführungsvariante kann vorgesehen sein, dass die Trägerstruktur und/oder die Reinigungskomponente zumindest einen nichtmetallischen Werkstoff aufweist oder daraus besteht, bevorzugt wobei der zumindest eine nichtmetallische Werkstoff zumindest eines der folgenden Materialien aufweist oder daraus besteht:
- Naturwerkstoff, besonders bevorzugt Grafit,
- Polymerwerkstoff, besonders bevorzugt Elastomer, Thermoplast, Duroplast, Copolymer,
- anorganischer Werkstoff, besonders bevorzugt Keramik oder Glas,
- Verbundwerkstoff, besonders bevorzugt verstärkter Faserverbundwerkstoff oder Schichtverbundwerkstoff.

Durch das Vermeiden von metallischen Werkstoffen bei der Reinigungseinheit können negative Einflüsse eines Magnetfeldes der medizinischen Bildgebungsvorrichtung wie beispielsweise Schäden an den Geräten, Verletzungsgefahr, höhere Aufwände für Installation, Wartung etc. vermieden oder zumindest verringert werden.

Gemäß einer bevorzugten Ausführungsform der Reinigungseinheit ist es vorgesehen, dass eine räumliche Ausdehnung, bevorzugt ein Außendurchmesser, der Reinigungseinheit
- bei nicht-befüllter Reinigungskomponente kleiner als die räumliche Ausdehnung, bevorzugt der Außendurchmesser, der Reinigungseinheit bei befüllter Reinigungskomponente ist und/oder
- bei nicht-befüllter Reinigungskomponente kleiner als eine räumliche Ausdehnung, bevorzugt ein Innendurchmesser, der Umhausung ist und/oder
- bei befüllter Reinigungskomponente mindestens gleich groß wie oder größer als eine räumlichen Ausdehnung, bevorzugt ein Innendurchmesser, der Umhausung ist.

Gemäß einer bevorzugten Ausführungsform der Reinigungseinheit ist es vorgesehen, dass das Druckmedium ein Gas oder eine Flüssigkeit, bevorzugt Druckluft, ist.

Ein Gas oder eine Flüssigkeit als Druckmedium können unterschiedliche Vorteile mit sich bringen. Beispielsweise sind Gase komprimierbar und können daher zu hohe Drücke besser ausgleichen. Flüssigkeiten sind kaum komprimierbar und übertragen daher Drücke nahezu ohne Druckverlust.

Gemäß einer bevorzugten Ausführungsform der Reinigungseinheit ist es vorgesehen, dass eine Verformungsvorrichtung vorgesehen ist, wobei die Verformungsvorrichtung einen Verformungsantrieb, ein Druckmedium und eine Verbindungsleitung zum, bevorzugt lösbaren oder unlösbaren, Verbinden der Reinigungskomponente mit dem Verformungsantrieb aufweist, wobei das Druckmedium mittels des Verformungsantriebs in die Reinigungskomponente einfüllbar und/oder aus der Reinigungskomponente entnehmbar ist, wobei die Reinigungskomponente durch Befüllen mit dem Druckmedium und/oder Entnehmen des Druckmediums verformbar ist.

Unter "lösbares Verbinden", "lösbar verbunden" und "lösbar verbindbar" kann verstanden werden, dass zumindest zwei ursprünglich voneinander getrennte Komponenten miteinander verbunden werden können, wobei dieses lösbare Verbinden anschließend zerstörungsfrei aufgelöst werden kann, sodass die zumindest zwei Komponenten wieder in ihrem ursprünglichen voneinander getrennten Zustand vorliegen, um daraufhin erneut verbunden werden zu können. "Lösbares Verbinden", "lösbar verbunden" und "lösbar verbindbar" kann daher auch als "zerstörungsfrei lösbares Verbinden", "zerstörungsfrei lösbar verbunden" und "zerstörungsfrei lösbar verbindbar" bezeichnet werden.

Unter "unlösbares Verbinden", "unlösbar verbunden" und "unlösbar verbindbar" kann verstanden werden, dass zumindest zwei ursprünglich voneinander getrennte Komponenten miteinander verbunden werden können, wobei dieses unlösbare Verbinden anschließend nicht aufgelöst werden kann, ohne dass die Integrität zumindest einer der zumindest zwei Komponenten beschädigt wird und/oder ohne dass ein erneutes Verbinden der zumindest zwei Komponenten erschwert oder ausgeschlossen wird. "Unlösbares Verbinden", "unlösbar verbunden" und "unlösbar verbindbar" kann daher auch als "zerstörungsfrei unlösbares Verbinden", "zerstörungsfrei unlösbar verbunden" und "zerstörungsfrei unlösbar verbindbar" bezeichnet werden.

In einer bevorzugten Ausführungsvariante kann vorgesehen sein, dass eine Verformungsvorrichtung einen Kompressor, bevorzugt einen Luftkompressor, aufweist, wobei der Kompressor das Druckmedium, bevorzugt Umgebungsluft, ansaugt, über eine Verbindungsleitung unter Druck zur Reinigungskomponente führt und die Reinigungskomponente somit befüllt.

Es kann eine Steuer- und Regelvorrichtung vorgesehen sein, mit der die Menge an Druckmedium und/oder der zu erreichende Druck innerhalb der befüllten Reinigungskomponente gesteuert und/oder geregelt werden kann.

Es kann zumindest ein Sensor, bevorzugt ein Drucksensor, vorgesehen sein, um den Zustand der befüllten und/oder nicht-befüllten Reinigungskomponente zu überwachen.

Eine Steuer- und Regelvorrichtung kann mit Sensoren verbunden sein.

Gemäß einer bevorzugten Ausführungsform der Reinigungseinheit ist es vorgesehen, dass die Reinigungskomponente einen Reinigungsgrundkörper und eine Reinigungsauflage aufweist, bevorzugt wobei die Reinigungsauflage entlang einer äußeren Kontur des Reinigungsgrundkörpers platziert ist.

In einer bevorzugten Ausführungsvariante kann vorgesehen sein, dass der Reinigungsgrundkörper ein schlauchförmiger Körper sein kann

In einer bevorzugten Ausführungsvariante kann vorgesehen sein, dass der Reinigungsgrundkörper eine flache Materialbahn, bevorzugt eine Membran, sein kann, wobei zumindest ein Ende, bevorzugt zumindest zwei Enden, der flachen Materialbahn, bevorzugt der Membran, mit der Trägerstruktur, bevorzugt entlang einer äußeren Kontur der Trägerstruktur, verbunden sein kann.

In einer bevorzugten Ausführungsvariante kann vorgesehen sein, dass der Reinigungsgrundkörper ein extrudiertes Profil und/oder ein extrudiertes Doppelprofil sein kann.

In einer bevorzugten Ausführungsvariante kann vorgesehen sein, dass die Reinigungsauflage einen Schaumstoff und/oder ein textiles Gebilde und/oder ein Nonwoven-Material aufweist.

In einer bevorzugten Ausführungsvariante kann vorgesehen sein, dass die Reinigungsauflage einen Schwamm und/oder einen Vliesstoff aufweist.

In einer bevorzugten Ausführungsvariante kann vorgesehen sein, dass die Reinigungsauflage eine Wischlippe aufweist oder daraus besteht.

In einer bevorzugten Ausführungsvariante kann vorgesehen sein, dass die Reinigungsauflage befeuchtet oder unbefeuchtet ist.

Gemäß einer bevorzugten Ausführungsform der Reinigungseinheit ist es vorgesehen, dass eine Platziervorrichtung vorgesehen ist, wobei die Reinigungsauflage mittels der Platziervorrichtung auf dem Reinigungsgrundkörper, bevorzugt entlang einer äußeren Kontur des Reinigungsgrundkörpers, platzierbar, bevorzugt aufspannbar, ist.

In einer bevorzugten Ausführungsvariante kann vorgesehen sein, dass die Platziervorrichtung einen Platzierantrieb und eine Verbindungsvorrichtung zum, bevorzugt lösbaren oder unlösbaren, Verbinden mit der Reinigungsauflage aufweist, wobei die mit der Verbindungsvorrichtung verbundene Reinigungsauflage durch den Platzierantrieb bis zu einer Betriebsstellung auf dem Reinigungsgrundkörper bewegbar, bevorzugt von einer Rolle abwickelbar, ist.

Durch die Platziervorrichtung kann das Platzieren, bevorzugt Aufspannen, der Reinigungsauflage zumindest teilweise automatisiert werden, wodurch der Wartungsaufwand der Reinigungseinheit verringert und/oder eine zuverlässigere Wartung erreicht werden kann.

In einer Betriebsstellung der Reinigungsauflage ist die Reinigungsauflage derart auf der Reinigungskomponente angeordnet, dass die Reinigungseinheit betriebsbereit ist und für das Reinigen der Umhausung verwendet werden kann. Bevorzugt kann vorgesehen sein, dass die Reinigungsauflage in der Betriebsstellung den Reinigungsgrundkörper teilweise oder vollständig bedeckt.

Durch die Platziervorrichtung kann ein Entfernen einer Reinigungsauflage von der Reinigungseinheit oder mit anderen Worten ein Deplatzieren einer Reinigungsauflage der Reinigungseinheit durchgeführt werden. In einer bevorzugten Ausführungsvariante kann somit ein Entfernen einer ersten, bevorzugt gebrauchten, Reinigungsauflage und/oder ein Platzieren einer zweiten, bevorzugt ungebrauchten, Reinigungsauflage erfolgen.

Gemäß einer bevorzugten Ausführungsform der Reinigungseinheit ist es vorgesehen, dass eine Aufbringvorrichtung zum Aufbringen eines Reinigungsmittels auf oder in die Reinigungsauflage vorgesehen ist, wobei das Reinigungsmittel vor, während und/oder nach dem Platzieren der Reinigungsauflage auf dem Reinigungsgrundkörper mittels der Aufbringvorrichtung aufbringbar ist.

Unter "Reinigungsmittel" kann hier ein allgemein bekanntes Mittel zum Säubern, beispielsweise Seife, ein Desinfektionsmittel, beispielsweise Alkohol, und/oder ein Sterilisationsmittel verstanden werden.

Durch die Aufbringvorrichtung kann das Aufbringen und/oder Einbringen von Reinigungsmittel auf und/oder in die Reinigungsauflage zumindest teilweise automatisiert werden, wodurch der Wartungsaufwand der Reinigungseinheit verringert und/oder eine zuverlässigere Wartung erreicht werden kann. Zudem kann somit eine höhere Flexibilität der Reinigung und eine höherer Anwenderfreundlichkeit erreicht werden. Beispielsweise kann durch die Wahl einer entsprechenden Aufbringvorrichtung der Zeitpunkt eines Befeuchten eines Vliestuches binnen eines Reinigungsverfahrens definiert werden. Zusätzlich oder stattdessen ist ein mehrmaliges Befeuchten eines Vliestuches möglich.

Gemäß einer bevorzugten Ausführungsform der Reinigungseinheit ist es vorgesehen, dass die Reinigungseinheit mittels einer Verfahrvorrichtung innerhalb der medizinischen Bildgebungsvorrichtung bewegbar ist, bevorzugt wobei die Verfahrvorrichtung ein beweglicher Patiententisch der medizinischen Bildgebungsvorrichtung oder eine von der medizinischen Bildgebungsvorrichtung getrennte Verfahrvorrichtung ist.

Im Fall eines beweglichen Patiententisches als Verfahrvorrichtung liegt der Vorteil darin, dass der Patiententisch eine bereits vorhandenen Verfahrvorrichtung einer medizinischen Bildgebungsvorrichtung darstellt und durch die Kopplung mit der Reinigungseinheit eine weitere Funktion, konkret der Reinigung der Umhausung, ermöglicht.

In einer bevorzugten Ausführungsvariante kann vorgesehen sein, dass die Reinigungseinheit, bevorzugt lösbar oder unlösbar, mit der Verfahrvorrichtung verbindbar oder verbunden ist. Mit Hilfe einer Kopplungsvorrichtung zum, bevorzugt lösbaren oder unlösbaren, Verbinden der Reinigungseinheit mit der Verfahrvorrichtung kann die Reinigungseinheit mittels der Verfahrvorrichtung in die Umhausung hinein und/oder innerhalb der Umhausung und/oder aus der Umhausung heraus bewegt werden. Nach oder bei dem Lösen der Reinigungseinheit von der Verfahrvorrichtung kann die Reinigungseinheit innerhalb der Umhausung oder bei oder in der Nachbarschaft einer Öffnung des Patientenaufnahmebereichs abgestellt werden.

In einer bevorzugten Ausführungsvariante kann vorgesehen sein, dass zumindest eine weitere Reinigungskomponente vorgesehen ist, bevorzugt wobei die zumindest eine weitere Reinigungskomponente, besonders bevorzugt unmittelbar, benachbart zu der Reinigungskomponente angeordnet ist.

In einer bevorzugten Ausführungsvariante kann vorgesehen sein, dass die zumindest eine weitere Reinigungskomponente entlang einer, bevorzugt äußeren, Kontur der Trägerstruktur oder entlang einer, bevorzugt äußeren, Kontur zumindest einer weiteren Trägerstruktur angeordnet ist.

Im Fall, dass die weitere Reinigungskomponente auf derselben Trägerstruktur wie die Reinigungskomponente angeordnet ist, kann die Breite der Trägerstruktur entsprechend an zwei oder mehrere Reinigungskomponenten angepasst sein.

Eine weitere Reinigungskomponente oder mit anderen Worten mindestens zwei Reinigungskomponenten einer einzigen Reinigungseinheit bietet den Vorteil, dass die Reinigungswirkung verbessert wird.

Hierbei kann zum Beispiel vorgesehen sein, dass bei starken Verschmutzungen zwei Reinigungskomponenten gleichzeitig zum Einsatz kommen. Es kann andererseits auch vorgesehen sein, dass die Umhausung in eine erste Richtung mit einer ersten Reinigungskomponente gereinigt werden kann und in eine zweite Richtung, konkret in die der ersten Richtung entgegengesetzte Richtung zurück zur Ausgangsposition, mit einer zweiten Reinigungskomponente gereinigt werden kann. Somit kann die Umhausung zweimal direkt hintereinander gereinigt werden, ohne dass eine Reinigungsauflage nach einem ersten Reinigen gewechselt werden muss oder eine der Reinigungsauflagen die Umhausung rekontaminiert.

Weiters wird Schutz begehrt für eine medizinische Bildgebungsvorrichtung mit einer Scannereinheit, einem von der Scannereinheit zumindest teilweise umgebenen Patientenaufnahmebereich, einer den Patientenaufnahmebereich umgebenden Umhausung und zumindest einer erfindungsgemäßen Reinigungseinheit, wobei die Reinigungseinheit innerhalb der Umhausung bewegbar ist.

Gemäß einer bevorzugten Ausführungsform der medizinischen Bildgebungsvorrichtung ist es vorgesehen, dass
- die nicht-befüllte Reinigungskomponente im Wesentlichen, bevorzugt vollständig, kontaktlos von der Umhausung umgeben ist und
- die befüllte Reinigungskomponente an der Umhausung, bevorzugt spaltfrei, besonders bevorzugt pressend, anliegt.

Gemäß einer bevorzugten Ausführungsform der medizinischen Bildgebungsvorrichtung ist es vorgesehen, dass die Reinigungseinheit, bevorzugt unlösbar oder lösbar, mit einer Verfahrvorrichtung, bevorzugt mit einem beweglichen Patiententisch der medizinischen Bildgebungsvorrichtung, verbunden oder verbindbar ist.

In einer bevorzugten Ausführungsvariante der medizinischen Bildgebungsvorrichtung kann vorgesehen sein, dass mittels der Verfahrvorrichtung die Reinigungseinheit in die Umhausung hineinbewegbar und/oder aus der Umhausung herausbewegbar und/oder innerhalb der Umhausung in zumindest eine Richtung, bevorzugt zwei Richtungen, bewegbar ist.

Mit Hilfe einer derartigen Verfahrvorrichtung kann zumindest eine Bewegung der Reinigungseinheit automatisiert werden. In einer besonders bevorzugten Ausführungsvariante kann vorgesehen sein, dass die Reinigungseinheit aus einer Parkstellung zumindest teilweise, bevorzugt vollständig, automatisiert in die Umhausung bewegt wird, dort die Reinigung der Umhausung durchgeführt wird und anschließend die Reinigungseinheit zurück in die Parkposition bewegt wird. Somit ist der Aufwand für eine Reinigung der Umhausung verringert.

Zum Bewegen der Reinigungseinheit kann eine Steuer- und Regelvorrichtung vorgesehen sein.

Weiters wird Schutz begehrt für ein Verfahren zum Reinigen einer erfindungsgemäßen medizinischen Bildgebungsvorrichtung mit einer erfindungsgemäßen Reinigungseinheit, folgende Verfahrensschritte aufweisend:
- Bereitstellen der Reinigungseinheit mit einer befüllten Reinigungskomponente und
- Reinigen einer Umhausung der medizinischen Bildgebungsvorrichtung mittels der befüllten Reinigungskomponente.

Gemäß einer bevorzugten Ausführungsform des Verfahrens zum Reinigen ist es vorgesehen, dass das Verfahren zumindest einen der folgenden Verfahrensschritte aufweist:
- Bewegen der Reinigungseinheit in die Umhausung hinein,
- Reinigen der Umhausung mittels der befüllten Reinigungskomponente entlang einer ersten Richtung und/oder entlang einer zweiten Richtung,
- Bereitstellen der Reinigungseinheit mit einer nicht-befüllten Reinigungskomponente,
- Kontaktloses Bewegen der nicht-befüllten Reinigungskomponente innerhalb der Umhausung, bevorzugt in eine erste Richtung und/oder eine zweite Richtung,
- Bewegen der Reinigungseinheit aus der Umhausung heraus.

In einer bevorzugten Ausführungsvariante des Verfahrens zum Reinigen kann vorgesehen sein, dass das Verfahren zumindest einen, bevorzugt alle der folgenden Verfahrensschritte aufweist:
- Bereitstellen der Reinigungseinheit mit einer befüllten Reinigungskomponente und mit einer weiteren nicht-befüllten Reinigungskomponente
- Reinigen der Umhausung mittels der befüllten Reinigungskomponente entlang einer ersten Richtung
- Kontaktloses Bewegen der weiteren nicht-befüllten Reinigungskomponente innerhalb der Umhausung in eine erste Richtung
- Bereitstellen der Reinigungseinheit mit der nicht-befüllten Reinigungskomponente und mit der weiteren befüllten Reinigungskomponente
- Kontaktloses Bewegen der nicht-befüllten Reinigungskomponente innerhalb der Umhausung in eine zweite Richtung
- Reinigen der Umhausung mittels der weiteren befüllten Reinigungskomponente entlang einer zweiten Richtung

Durch dieses bevorzugte Ausführungsbeispiel eines Verfahrens können, wie bereits oben beschrieben, zumindest zwei Reinigungskomponenten innerhalb eines Reinigungszyklus benützt werden, wobei die zumindest zwei Reinigungskomponenten gleichzeitig oder hintereinander eingesetzt werden können, ohne dass eine Reinigungsauflage gewechselt werden muss oder die Gefahr einer Rekontamination besteht.

In einer bevorzugten Ausführungsvariante des Verfahrens zum Reinigen kann vorgesehen sein, dass das Verfahren zumindest einen, bevorzugt alle, der folgenden Verfahrensschritte aufweisend:
- Bereitstellen der Reinigungseinheit mit einer befüllten Reinigungskomponente,
- Einschnüren einer, bevorzugt befüllten, Reinigungskomponente, bevorzugt entlang einer äußeren Kontur der Reinigungskomponente und/oder wobei der Verlauf der Einschnürung zu einer ersten Richtung geneigt, besonders bevorzugt orthogonal ist, zum Bereitstellen einer befüllten Reinigungskomponente mit zumindest zwei Kontaktflächen, wobei zumindest eine der zumindest zwei Kontaktflächen bei befüllter Reinigungskomponente an der Umhausung, bevorzugt pressend, anliegt,
- Reinigen der Umhausung mittels zumindest einer der zumindest zwei Kontaktflächen der befüllten und eingeschnürten Reinigungskomponente entlang einer ersten Richtung,
- Bereitstellen der Reinigungseinheit mit der nicht-befüllten Reinigungskomponente,
- Kontaktloses Bewegen der nicht-befüllten Reinigungskomponente innerhalb der Umhausung in eine zweite Richtung.

Durch dieses bevorzugte Ausführungsbeispiel eines Verfahrens können zumindest zwei Reinigungsschritte beim Bewegen der Reinigungseinheit innerhalb der Umhausung in eine erste Richtung erfolgen, ohne dass es zu einer Rekontamination kommt.

Beispielsweise kann die Einschnürung einer einzelnen Reinigungskomponente, konkret einer mit der Trägerstruktur verbundenen Membran als Reinigungsgrundkörper und eines auf der Membran angeordneten Vliesstoffes als Reinigungsauflage, dazu führen, dass die Reinigungskomponente zwei Wulste ausbildet. Diese beiden Wulste können entlang einer äußeren Kontur der Reinigungskomponente verlaufen und der Verlauf der Wulste kann geneigt, bevorzugt orthogonal, zu einer Bewegungsrichtung der Reinigungseinheit innerhalb der Umhausung ausgerichtet sein. Auf diese Weise kann eine erste Wulst in eine erste Richtung eine Vorreinigung durchführen, während die zweite Wulst direkt nachfolgend in die erste Richtung eine Nachreinigung durchführt. Wenn die Reinigungseinheit die Bewegung und damit die Reinigung in die erste Richtung beendet, kann die Reinigungskomponente in den nicht-befüllten Zustand versetzt werden, wodurch beide Wulste der Reinigungskomponente die Umhausung nicht mehr kontaktieren und in die zweite Richtung zurückgefahren werden können.

Es kann vorgesehen sein, dass bei einer befüllten und eingeschnürten Reinigungskomponente mit zumindest zwei Kontaktflächen zumindest eine Kontaktfläche die Umhausung kontaktiert und zumindest eine Kontaktfläche die Umhausung nicht kontaktiert.

Auf diese Weise kann beim Bewegen der Reinigungseinheit in eine erste Richtung die Umhausung mit zumindest einer ersten Kontaktfläche gereinigt werden, während zumindest eine zweite Kontaktfläche die Umhausung nicht kontaktiert. Anschließend kann vorgesehen sein, dass die Befüllung und/oder die Einschnürung der Reinigungskomponente derart manipuliert wird, dass beim Bewegen der Reinigungseinheit in eine zweite Richtung die Umhausung mit der zumindest einen zweiten Kontaktfläche gereinigt wird, während die zumindest eine erste Kontaktfläche die Umhausung nicht mehr kontaktiert.

In einer bevorzugten Ausführungsvariante kann vorgesehen sein, dass ein Reinigen einer Umhausung der medizinischen Bildgebungsvorrichtung zusätzlich mittels einer Bestrahlungsvorrichtung, bevorzugt mittels UV-bestrahlung, vorgesehen ist.

Die Kombination eines Reinigens einer Umhausung der medizinischen Bildgebungsvorrichtung mittels der befüllten Reinigungskomponente und einer Bestrahlungsvorrichtung kann eine noch zuverlässigere Reinigung ermöglichen.

Weitere Einzelheiten und Vorteile bevorzugter Ausgestaltungsbeispiele der Erfindung werden anhand der Figurenbeschreibung unter Bezugnahme auf die Zeichnungen im Folgenden näher erläutert. Darin zeigen:
- Fig. 1:: eine schematische, perspektivische Ansicht einer medizinischen Bildgebungsvorrichtung;
- Fig. 2:: eine schematische, perspektivische Ansicht in die Umhausung der medizinischen Bildgebungsvorrichtung aus Fig. 1 mit einer erfindungsgemäßen Reinigungseinheit;
- Fig. 3:: eine schematische, perspektivische Ansicht einer ersten Ausführungsvariante der Reinigungseinheit aus Fig. 2;
- Fig. 4:: eine schematische, perspektivische Ansicht einer zweiten Ausführungsvariante der Reinigungseinheit aus Fig. 2;
- Fig. 5 bis 9:: verschiedene Ansichten der Reinigungseinheit aus Fig. 3 in einem befüllten oder nicht-befüllten Zustand;
- Fig. 10 bis 12:: verschiedene Vorderansichten einer Umhausung mit der darin angeordneten Reinigungseinheit aus Fig. 3 in einem befüllten oder nicht-befüllten Zustand.

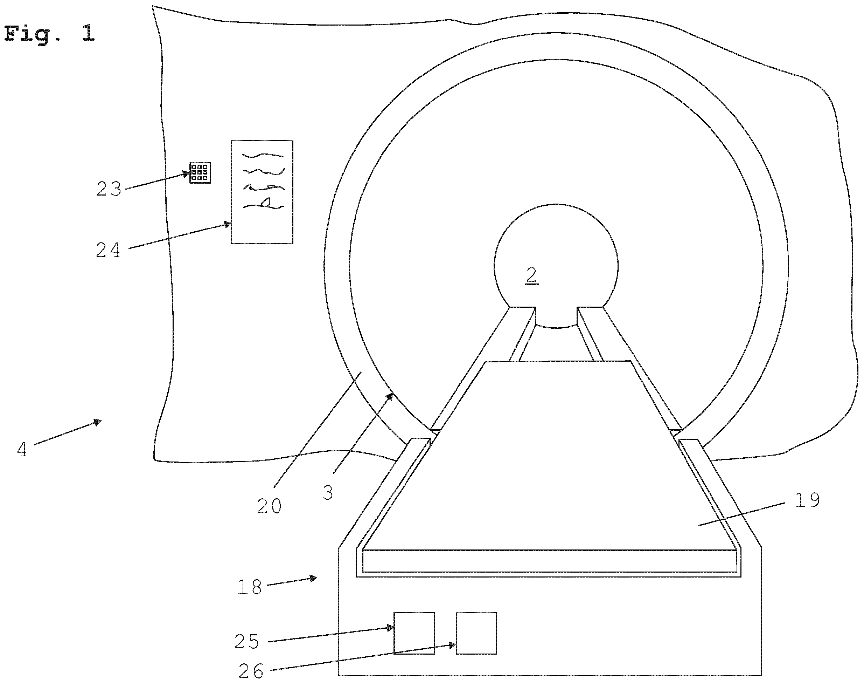

Fig. 1 zeigt eine schematische, perspektivische Ansicht einer medizinischen Bildgebungsvorrichtung 4.

Die medizinische Bildgebungsvorrichtung 4 in Fig. 1 ist eine medizinische Bildgebungsvorrichtung 4 mit einer Scannereinheit 20, einem von der Scannereinheit 20 zumindest teilweise umgebenen Patientenaufnahmebereich 2, einer den Patientenaufnahmebereich 2 umgebenden Umhausung 3.

Eine solche medizinische Bildgebungsvorrichtung 4 kann zumindest eine erfindungsgemäße Reinigungseinheit 1 aufweisen, wobei die Reinigungseinheit 1 innerhalb der Umhausung 3 bewegbar sein kann.

Eine solche medizinische Bildgebungsvorrichtung 4 kann eine Bedienungsvorrichtung 23 und eine Ausgabevorrichtung 24 aufweisen.

Eine solche medizinische Bildgebungsvorrichtung 4 kann eine Verfahrvorrichtung 18 aufweisen. Die Verfahrvorrichtung 18 kann einen beweglichen Patiententisch 19, einen Verfahrantrieb 25 und eine Steuer- und Regeleinheit 26 aufweisen.

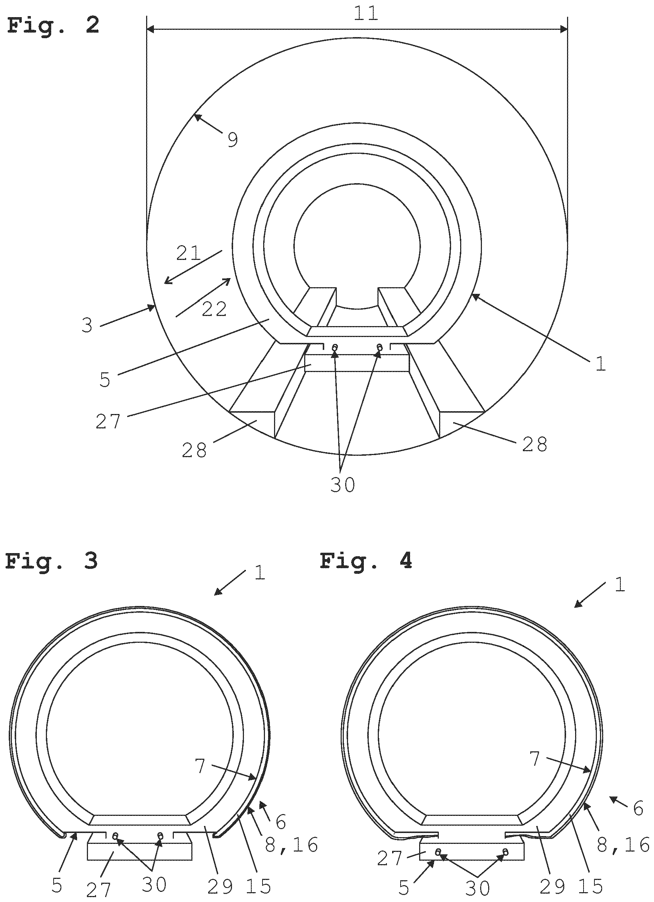

Fig. 2 zeigt eine schematische, perspektivische Ansicht in die Umhausung 3 der medizinischen Bildgebungsvorrichtung 4 aus Fig. 1 mit einer erfindungsgemäßen Reinigungseinheit 1.

Fig. 2 zeigt eine Reinigungseinheit 1 zum Reinigen einer einen Patientenaufnahmebereich 2 umgebenden Umhausung 3 einer medizinischen Bildgebungsvorrichtung 4, mit einer Trägerstruktur 5 und mit einer Reinigungskomponente 6, wobei die Reinigungskomponente 6 der in die Umhausung 3 eingeführten Reinigungseinheit 1 zwischen der Trägerstruktur 5 und der Umhausung 3 angeordnet ist, dadurch gekennzeichnet, dass die Reinigungskomponente 6 in einem mit einem Druckmedium befüllten Zustand an der Umhausung 3, bevorzugt pressend, anliegt.

Die Reinigungskomponente 6 ist in Fig. 2 aus Gründen der Übersichtlichkeit weggelassen.

Die Trägerstruktur 5 der Reinigungseinheit 1 kann ein Basisteil 27 und ein Ringteil 29 aufweisen. Es ist aber auch denkbar, dass die Trägerstruktur kein Basisteil 27 aufweist.

Die Reinigungseinheit kann zumindest eine Kopplungsvorrichtung 30 zum, bevorzugt lösbaren oder unlösbaren, Verbinden mit einer Verfahrvorrichtung 18 aufweisen.

Es kann zumindest eine Kopplungsvorrichtung 30 auf der Trägerstruktur, bevorzugt auf dem Basisteil 27 und/oder auf dem Ringteil 29 vorgesehen sein.

Die medizinische Bildgebungsvorrichtung 4 kann zwei Lichtleisten 28 aufweisen.

Die Umhausung 3 kann eine innere Kontur 9 aufweisen. Im Fall einer hohlzylinderförmigen Umhausung 3, wie in Fig. 2 dargestellt, entspricht die Innenseite der Umhausung 3 einer Mantelfläche eines Zylinders. Eine innere Kontur 9 der Umhausung kann somit kreisförmig oder kreisbogenförmig sein.

Die Reinigungseinheit 1 kann in zumindest eine Richtung, bevorzugt in eine erste Richtung 21 und/oder in eine zweite Richtung 22, innerhalb der Umhausung 3 bewegt werden.

Fig. 3 zeigt eine schematische, perspektivische Ansicht einer ersten Ausführungsvariante der Reinigungseinheit 1 aus Fig. 2.

Die Reinigungseinheit 1 kann eine Trägerstruktur 5 und eine Reinigungskomponente 6 aufweisen.

Die Trägerstruktur 5 kann ein Basisteil 27 und ein Ringteil 29 aufweisen, wobei das Ringteil 29 eine Kooplungsvorrichtung 30 zum, bevorzugt lösbaren oder unlösbaren, Verbinden mit einer Verfahrvorrichtung 18 aufweisen kann.

Das Ringteil 29 der Trägerstruktur 5 in Fig. 3 und 4 weist eine äußere Kontur 7 auf, welche nahezu einen vollständigen Kreis, konkret einen Kreisbogen mit einem Winkel über 180°, beschreibt. Es kann vorgesehen sein, dass die Trägerstruktur 5 eine äußere Kontur 7 aufweist, welche einen vollständigen Kreis oder einen unvollständigen Kreis, konkret einen Kreisbogen, aufweist.

Die Reinigungskomponente 6 kann einen Reinigungsgrundkörper 15 und eine Reinigungsauflage 16 aufweisen, wobei der Reinigungsgrundkörper 15 zwischen der Trägerstruktur 5, konkret dem Ringteil 29 und/oder der äußeren Kontur 7 der Trägerstruktur 5, und der Reinigungsauflage 16 angeordnet sein kann.

Die Reinigungsauflage 16 kann flexibel ausgeführt sein, sodass sich die Reinigungsauflage 16 der Form des Reinigungsgrundkörpers 15 anpasst. In Fig. 3 und 4 entspricht die Form einer äußeren Kontur des Reinigungsgrundkörpers 15 im Wesentlichen der Form der äußeren Kontur der auf dem Reinigungsgrundkörper 15 angeordneten Reinigungsauflage 16.

In Fig. 3 und 4 entspricht die Form einer äußeren Kontur 7 der Trägerstruktur 5 im Wesentlichen der Form der äußeren Kontur 8 der Reinigungskomponente 6, konkret in Fig. 3 und 4 der äußeren Kontur der auf der Reinigungskomponente 6 angeordneten Reinigungsauflage 16.

In Fig. 3 und 4 ist die Form der äußeren Kontur 8 der Reinigungskomponente 6 im Wesentlichen kreisförmig, konkret kreisbogenförmig.

Fig. 4 zeigt eine schematische, perspektivische Ansicht einer zweiten Ausführungsvariante der Reinigungseinheit 1 aus Fig. 2.

Bisher Gesagtes zu Fig. 3 gilt analog für Fig. 4.

Fig. 3 und Fig. 4 unterscheiden sich dahingehend,
- dass die Reinigungskomponente 6 in Fig. 4 über den Kreisbogen des Ringteils 29 hinaus bis zum Basisteil 27 weiterverläuft und
- dass die Kopplungsvorrichtungen 30 in Fig. 4 auf dem Basisteil 27 anstatt auf dem Ringteil 29 angeordnet sind.

In Fig. 4 ist die Reinigungsauflage 16 ebenso weitergeführt wie die Reinigungsgrundkörper 15.

Auf diese Weise kann sowohl die Innenseite der Umhausung 3 als auch zumindest eine Seite der Lichtleisten 28, konkret die Oberseite der Lichtleiste 28, gereinigt werden.

Die Position der Kopplungsvorrichtungen 30 kann auf dem Basisteil 27 und/oder auf dem Ringteil 29 vorgesehen sein. Für die Fälle, dass die Verfahrvorrichtung 18 zum Bewegen der Reinigungseinheit 1 der Patiententisch der medizinischen Bildgebungsvorrichtung ist oder dass die Verfahrvorrichtung eine von der medizinischen Bildgebungsvorrichtung getrennte Vorrichtung ist, können unterschiedliche Positionen der Kopplungsvorrichtung vorteilhaft sein.

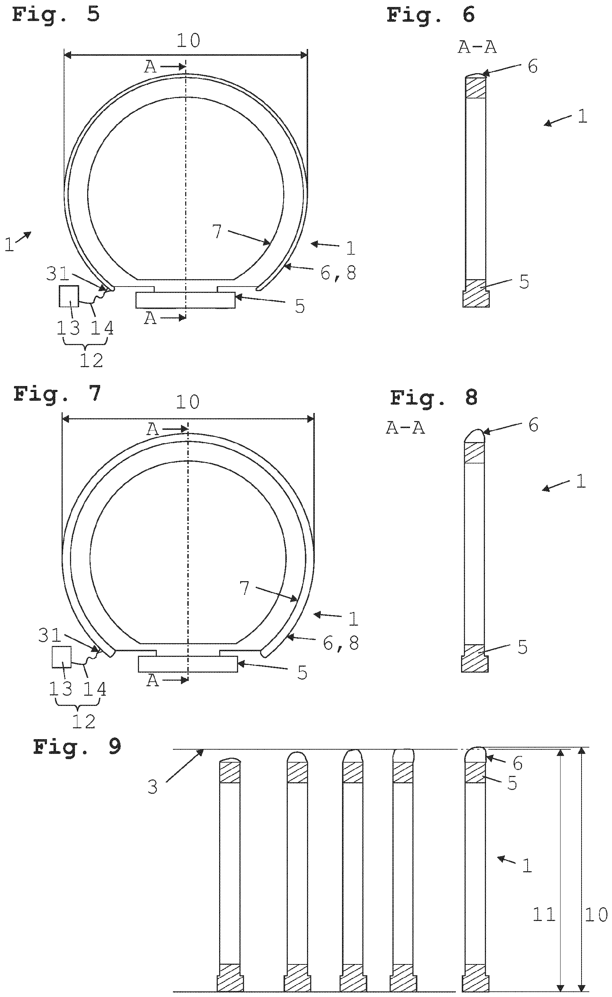

Für die restlichen Ausführungsformen in den Fig. 5 bis 12 werden die Reinigungsauflage 16 und die Kopplungsvorrichtungen 30 aus Gründen der Übersichtlichkeit nicht mehr explizit dargestellt. Die Reinigungsauflage 16 und die Kopplungsvorrichtungen 30 können dennoch vorgesehen sein.

Für die restlichen Ausführungsformen in den Fig. 5 bis 12 wird die bereits beschriebene Reinigungskomponente 6 aus Fig. 3 herangezogen, wobei die in Fig. 4 beschriebene Reinigungskomponente 6 zusätzlich und/oder stattdessen Anwendung finden kann.

Es sei an dieser Stelle angemerkt, dass die Reinigungseinheit 1 zumindest zwei gleichartige Reinigungskomponenten 6 oder zumindest zwei verschiedenartige, beispielsweise die Reinigungskomponente 6 aus Fig. 3 und die Reinigungskomponente aus Fig. 4, aufweisen kann.

Fig. 5 zeigt eine Vorderansicht der Reinigungseinheit 1 aus Fig. 3 in einem nicht-befüllten Zustand.

In Fig. 5 weist die Reinigungskomponente 6 gar kein oder nur wenig Druckmedium auf. Wichtig ist, dass die äußere Kontur 8 der Reinigungskomponente 6 eine räumliche Ausdehnung 10 aufweist, welche kleiner als eine räumliche Ausdehnung 10 der Reinigungskomponente 6 im befüllten Zustand und/oder kleiner als eine räumliche Ausdehnung 11 der Umhausung 3 ist. Mit anderen Worten, wichtig ist, dass die äußere Kontur 8 der Reinigungskomponente 6 im nicht-befüllten Zustand die Umhausung nicht kontaktiert.

Es kann eine Verformungsvorrichtung 12 vorgesehen sein, wobei die Verformungsvorrichtung 12 einen Verformungsantrieb 13 und eine Verbindungsleitung 14 aufweist. Die Verbindungsleitung 14 dient zum Verbinden des Verformungsantriebs 13 mit der Reinigungseinheit 1, konkret der Reinigungskomponente 6. Die Reinigungseinheit 1 kann einen Anschluss 31 für die Verbindungsleitung 14 aufweisen.

Der Anschluss kann verschließbar sein und entweder manuell oder automatisch geöffnet und/oder geschlossen werden.

Der Verformungsantrieb 13 kann beispielsweise ein Luftkompressor sein, welcher Luft aus der Umgebung durch die Verbindungsleitung 14 fördert und somit die Reinigungskomponente 6 mit Druckluft befüllt.

Fig. 6 zeigt eine Schnittdarstellung A-A der Reinigungseinheit 1 aus Fig. 3 in einem nicht-befüllten Zustand.

In Fig. 6 ist klar erkenntlich, dass die Reinigungskomponente 6 wenig bis gar kein Druckmedium einschließt.

Fig. 7 zeigt eine Vorderansicht der Reinigungseinheit aus Fig. 3 in einem befüllten Zustand.

In Fig. 7 ist klar erkenntlich, dass die räumliche Ausdehnung 10 der Reinigungskomponente 6 grösser als die räumlichen Ausdehnung 10 der Reinigungskomponente 6 in Fig. 5 ist. Dieser befüllte Zustand der Reinigungskomponente 6 kann durch Befüllen der Reinigungskomponente 6 mit einem Druckmedium durch die Verformungsvorrichtung 12 erreicht werden.

Fig. 8 zeigt eine Schnittdarstellung A-A der Reinigungseinheit aus Fig. 3 in einem befüllten Zustand.

In Fig. 8 ist klar erkenntlich, dass die Reinigungskomponente 6 mehr Druckmedium als in Fig. 6 einschließt.

Fig. 9 zeigt verschiedene Schnittdarstellung A-A der Reinigungseinheit 1 aus Fig. 3 mit verschiedenen Graden der Befüllung mit dem Druckmedium.

Die verschiedenen Grade der Befüllung der Reinigungskomponente 6 werden in Fig. 9 von links nach rechts dargestellt, wobei der Grad der Befüllung von links nach rechts zunimmt.

Die erste Darstellungen in Fig. 9 zeigt eine Reinigungskomponente 6 im nicht-befüllten Zustand und die zweite Darstellung einer Reinigungskomponente 6 mit einer geringen Menge an Druckmedium, welche durch die Reinigungskomponente 6 eingeschlossen wird, zeigt die Reinigungskomponente 6 im teilweise befüllten Zustand. Bei keiner dieser beiden Darstellungen einer nicht-befüllten oder teilweise befüllten Reinigungskomponente kontaktiert die Reinigungskomponente 6 die Umhausung 3.

Die dritte Darstellung in Fig. 9 zeigt einen ersten befüllten Zustand der Reinigungskomponente 6, in dem die befüllte Reinigungskomponente 6 die Umhausung 3 kontaktiert.

Die vierte Darstellung in Fig. 9 zeigt einen zweiten befüllten Zustand der Reinigungskomponente 6, wobei mehr Druckmedium von der Reinigungskomponente 6 in der vierten Darstellung als in der dritten Darstellung eingeschlossen wird. Somit ist die Kontaktfläche der Reinigungskomponente 6 der vierten Darstellung mit der Umhausung 3 grösser als in der dritten Darstellung in Fig. 9.

Wie in Fig. 9 dargestellt kann durch den höheren Grad an Befüllung, siehe hierfür die dritte und vierte Darstellung, der Anpressdruck der Reinigungskomponente 6 gegen die Umhausung 3 erhöht und eine bessere Abdichtung ermöglicht werden, um eine noch zuverlässigere Reinigung zu erreichen.

Die fünfte Darstellung in Fig. 9 zeigt, dass die räumliche Ausdehnung 10 der befüllten Reinigungskomponente 6 ohne den Widerstand der Umhausung 3 grösser ist als die räumliche Ausdehnung 11 der Umhausung 3.

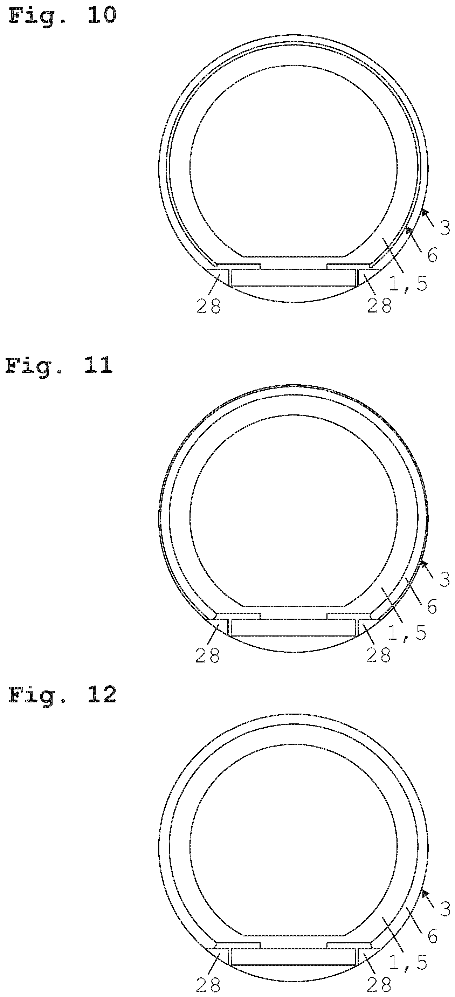

Fig. 10 zeigt eine Vorderansicht einer Umhausung 3 mit der darin angeordneten Reinigungseinheit 1 aus Fig. 3 in einem nicht-befüllten Zustand.

In Fig. 10 ist zu erkennen, dass die Reinigungskomponente 6 in einem nicht-befüllten Zustand vorliegt und somit kein Kontaktieren mit der Umhausung 3 realisiert ist.

Fig. 11 zeigt eine Vorderansicht einer Umhausung 3 mit der darin angeordneten Reinigungseinheit 1 aus Fig. 3 in einem teilweise befüllten Zustand kurz vor einem befüllten Zustand.

In Fig. 11 ist zu erkennen, dass die Reinigungskomponente 6 im Vergleich zur Fig. 10 bereits mit einer geringen Menge an Druckmedium befüllt ist, jedoch liegt die Reinigungskomponente 6 nach wie vor in einem nur teilweise befüllten Zustand vor, weshalb die Umhausung 3 nicht kontaktiert wird.

Es sei an dieser Stelle angemerkt, dass in Fig. 11 zwar nicht die Umhausung 3 dafür aber die Lichtleisten 28 durch die Reinigungskomponente 6 kontaktiert werden.

Fig. 12 zeigt eine Vorderansicht einer Umhausung mit der darin angeordneten Reinigungseinheit aus Fig. 3 in einem befüllten Zustand.

In Fig. 12 ist zu erkennen, dass die Reinigungskomponente 6 im Vergleich zu den Figuren 10 und 11 eine ausreichende Menge an Druckmedium einschließt und sich somit in einem befüllten Zustand befindet. In dem dargestellten befüllten Zustand der Reinigungskomponente 6 wird die Umhausung 3 durch die befüllte Reinigungskomponente 6 kontaktiert.

Wie in Fig. 12 dargestellt, kann bevorzugt vorgesehen sein, dass die befüllte Reinigungskomponente 6 eine spaltfreie oder mit anderen Worten lückenlose Kontaktierung mit der Umhausung 3 realisiert.

Es sei an dieser Stelle angemerkt, dass in Fig. 12 neben der Umhausung 3 auch die Lichtleisten 28 durch die Reinigungskomponente 6 kontaktiert werden. Jedoch wird bei dieser Ausführungsvariante nicht die komplette Oberseite der Lichtleisten 27 kontaktiert. Im Fall, dass die Ausführungsvariante aus Fig. 4 zum Einsatz kommt, bei der die Reinigungskomponente 6 bis zum Ansatz des Basisteils 27 reicht, besteht der Vorteil, neben der Umhausung 3 auch die komplette Oberseite der Lichtleisten 28 reinigen zu können. Mit Hilfe der Ausführungsvariante aus Fig. 4 überdeckt die Reinigungskomponente 6 auch den Spalt, erkennbar in Fig. 10 bis 12, der zwischen den Lichtleisten 28 und dem Basisteil 27 ist.

### Bezugszeichenliste

- 1: Reinigungseinheit
- 2: Patientenaufnahmeberei ch
- 3: Umhausung
- 4: medizinische Bildgebungsvorrichtung
- 5: Trägerstruktur
- 6: Reinigungskomponente
- 7: Kontur, bevorzugt äußere Kontur, der Trägerstruktur
- 8: äußere Kontur der Reinigungskomponente
- 9: innere Kontur der Umhausung
- 10: räumliche Ausdehnung der Reinigungseinheit
- 11: räumliche Ausdehnung der Umhausung
- 12: Verformungsvorrichtung
- 13: Verformungsantrieb
- 14: Verbindungsleitung
- 15: Reinigungsgrundkörper
- 16: Reinigungsauflage
- 17: äußere Kontur der Reinigungsgrundkörper
- 18: Verfahrvorrichtung
- 19: Patiententisch
- 20: Scannereinheit
- 21: erste Richtung
- 22: zweite Richtung
- 23: Bedienungsvorrichtung
- 24: Ausgabevorrichtung
- 25: Verfahrantrieb
- 26: Steuer- und Regeleinheit der Verfahrvorrichtung
- 27: Basisteil
- 28: Lichtleiste
- 29: Ringteil
- 30: Kopplungsvorrichtung
- 31: Anschluss

## Patentansprüche

1. Reinigungseinheit (1) zum Reinigen einer einen Patientenaufnahmebereich (2) umgebenden Umhausung (3) einer medizinischen Bildgebungsvorrichtung (4), mit einer Trägerstruktur (5) und mit einer Reinigungskomponente (6), wobei die Reinigungskomponente (6) der in die Umhausung (3) eingeführten Reinigungseinheit (1) zwischen der Trägerstruktur (5) und der Umhausung (3) angeordnet ist, **dadurch gekennzeichnet, dass** die Reinigungskomponente (6) in einem mit einem Druckmedium befüllten Zustand an der Umhausung (3), bevorzugt pressend, anliegt.

2. Reinigungseinheit (1) zum Reinigen einer einen Patientenaufnahmebereich (2) umgebenden Umhausung (3) einer medizinischen Bildgebungsvorrichtung (4), mit einer Trägerstruktur (5) und mit einer Reinigungskomponente (6), wobei die Reinigungskomponente (6) entlang einer, bevorzugt äußeren, Kontur (7) der Trägerstruktur (5) angeordnet ist, **dadurch gekennzeichnet, dass** die Reinigungskomponente (6) durch Befüllen mit einem Druckmedium verformbar ist.

3. Reinigungseinheit (1) nach Anspruch 1 oder 2, **dadurch gekennzeichnet, dass** eine äußere Kontur (7,8) der Trägerstruktur (5) und/oder der, bevorzugt befüllten, Reinigungskomponente (6) korrespondierend zu einer inneren Kontur (9) der Umhausung (3), bevorzugt gebogen, besonders bevorzugt kreisförmig, ist.

4. Reinigungseinheit (1) nach einem der vorangegangenen Ansprüche, **dadurch gekennzeichnet, dass** eine räumliche Ausdehnung (10), bevorzugt ein Außendurchmesser, der Reinigungseinheit (1)
- bei nicht-befüllter Reinigungskomponente (6) kleiner als die räumliche Ausdehnung (10), bevorzugt der Außendurchmesser, der Reinigungseinheit (1) bei befüllter Reinigungskomponente (6) ist und/oder
- bei nicht-befüllter Reinigungskomponente (6) kleiner als eine räumliche Ausdehnung (11), bevorzugt ein Innendurchmesser, der Umhausung (3) ist und/oder
- bei befüllter Reinigungskomponente (6) mindestens gleich groß wie oder größer als eine räumlichen Ausdehnung (11), bevorzugt ein Innendurchmesser, der Umhausung (3) ist.

5. Reinigungseinheit (1) nach einem der vorangegangenen Ansprüche, **dadurch gekennzeichnet, dass** das Druckmedium ein Gas oder eine Flüssigkeit, bevorzugt Druckluft, ist.

6. Reinigungseinheit (1) nach einem der vorangegangenen Ansprüche, **dadurch gekennzeichnet, dass** eine Verformungsvorrichtung (12) vorgesehen ist, wobei die Verformungsvorrichtung (12) einen Verformungsantrieb (13), ein Druckmedium und eine Verbindungsleitung (14) zum, bevorzugt lösbaren oder unlösbaren, Verbinden der Reinigungskomponente (6) mit dem Verformungsantrieb (13) aufweist, wobei das Druckmedium mittels des Verformungsantriebs (13) in die Reinigungskomponente (6) einfüllbar und/oder aus der Reinigungskomponente (6) entnehmbar ist, wobei die Reinigungskomponente (6) durch Befüllen mit dem Druckmedium und/oder Entnehmen des Druckmediums verformbar ist.

7. Reinigungseinheit (1) nach einem der vorangegangenen Ansprüche, **dadurch gekennzeichnet, dass** die Reinigungskomponente (6) einen Reinigungsgrundkörper (15) und eine Reinigungsauflage (16) aufweist, bevorzugt wobei die Reinigungsauflage (16) entlang einer äußeren Kontur (17) des Reinigungsgrundkörpers (15) platziert ist.

8. Reinigungseinheit (1) nach einem der vorangegangenen Ansprüche, **dadurch gekennzeichnet, dass** eine Platziervorrichtung vorgesehen ist, wobei die Reinigungsauflage (16) mittels der Platziervorrichtung auf den Reinigungsgrundkörper (15), bevorzugt entlang einer äußeren Kontur (17) des Reinigungsgrundkörpers (15), platzierbar, bevorzugt aufspannbar, ist.

9. Reinigungseinheit (1) nach einem der vorangegangenen Ansprüche, **dadurch gekennzeichnet, dass** eine Aufbringvorrichtung zum Aufbringen eines Reinigungsmittels auf oder in die Reinigungsauflage (16) vorgesehen ist, wobei das Reinigungsmittel vor, während und/oder nach dem Platzieren der Reinigungsauflage (16) auf den Reinigungsgrundkörper (15) mittels der Aufbringvorrichtung aufbringbar ist.

10. Reinigungseinheit (1) nach einem der vorangegangenen Ansprüche, **dadurch gekennzeichnet, dass** die Reinigungseinheit (1) mittels einer Verfahrvorrichtung (18) innerhalb der medizinischen Bildgebungsvorrichtung (4) bewegbar ist, bevorzugt wobei die Verfahrvorrichtung (18) ein beweglicher Patiententisch (19) der medizinischen Bildgebungsvorrichtung (4) oder eine von der medizinischen Bildgebungsvorrichtung (4) getrennte Verfahrvorrichtung (18) ist.

11. Medizinische Bildgebungsvorrichtung (4) mit einer Scannereinheit (20), einem von der Scannereinheit 20 zumindest teilweise umgebenen Patientenaufnahmebereich (2), einer den Patientenaufnahmebereich (2) umgebenden Umhausung (3) und zumindest einer Reinigungseinheit (1) nach einem der vorhergehenden Ansprüche 1 bis 10, wobei die Reinigungseinheit (1) innerhalb der Umhausung (3) bewegbar ist.

12. Medizinische Bildgebungsvorrichtung (4) nach dem vorangegangenen Anspruch 11, **dadurch gekennzeichnet, dass**
- die nicht-befüllte Reinigungskomponente (6) im Wesentlichen, bevorzugt vollständig, kontaktlos von der Umhausung (3) umgeben ist und
- die befüllte Reinigungskomponente (6) an der Umhausung (3), bevorzugt spaltfrei, besonders bevorzugt pressend, anliegt.

13. Medizinische Bildgebungsvorrichtung (4) nach einem der vorangegangenen Ansprüche 11 oder 12, **dadurch gekennzeichnet, dass** die Reinigungseinheit (1), bevorzugt unlösbar oder lösbar, mit einer Verfahrvorrichtung (18), bevorzugt mit einem beweglichen Patiententisch (19) der medizinischen Bildgebungsvorrichtung (4), verbunden oder verbindbar ist.

14. Verfahren zum Reinigen einer medizinischen Bildgebungsvorrichtung (4) nach einem der vorhergehenden Ansprüche 11 bis 13 mit einer Reinigungseinheit (1) nach einem der Ansprüche 1 bis 10, folgende Verfahrensschritte aufweisend:
- Bereitstellen der Reinigungseinheit (1) mit einer befüllten Reinigungskomponente (6) und
- Reinigen einer Umhausung (3) der medizinischen Bildgebungsvorrichtung (4) mittels der befüllten Reinigungskomponente (6).

15. Verfahren nach dem vorangegangenen Anspruch 14, zumindest einen der folgenden Verfahrensschritte aufweisend:
- Bewegen der Reinigungseinheit (1) in die Umhausung (3) hinein,
- Reinigen der Umhausung (3) mittels der befüllten Reinigungskomponente (6) entlang einer ersten Richtung (21) und/oder entlang einer zweiten Richtung (22),
- Bereitstellen der Reinigungseinheit (1) mit einer nicht-befüllten Reinigungskomponente (6),
- Kontaktloses Bewegen der nicht-befüllten Reinigungskomponente (6) innerhalb der Umhausung (3), bevorzugt in eine erste Richtung (21) und/oder eine zweite Richtung (22),
- Bewegen der Reinigungseinheit (1) aus der Umhausung (3) heraus.
